# EUROPÄISCHE PATENTANMELDUNG

(11) **EP 1 063 241 A1**
(43) Veröffentlichungstag der Anmeldung: **27.12.2000**
(21) Anmeldenummer: 00121934.4
(22) Anmeldetag: 18.10.1994
(51) Int. Cl.: C08B 37/16, A61K 47/48, A61K 31/385, A61K 31/19

(54) **Arzneimittelzubereitungen enthaltend Thioctsäure oder Dihydroliponsäure in Form von Einschlussverbindungen mit Cyclodextrinen oder Cyclodextrinderivaten und in Form von Granulaten, Kau- oder Brausetabletten**

(30) Priorität: 11.11.1993 DE 4338508
(62) Teilanmeldung aus: 94116380.0
(71) Anmelder: ASTA Medica Aktiengesellschaft, 01277 Dresden (DE)
(72) Erfinder: Hettche, Helmut, Dr., 63128 Dietzenbach (DE)

(57) **Zusammenfassung**

Der unangenehme Geschmack der Thioctsäure oder der Dihydroliponsäure läßt sich durch Einbettung in Cyclodextrine kaschieren. Arzneiformen werden beschrieben.
Neu sind die Einschlußverbindungen aus enantiomerenreiner Dihydroliponsäure, Thioctsäure und Cyclodextrinen. Sie eignen sich zur Herstellung von Arzneimitteln.

## Beschreibung

Die vorliegende Erfindung betrifft Arzneimittelformulierungen, die Thioctsäure oder Dihydroliponsäure enthalten, wobei die Thioctsäure oder Dihydroliponsäure als Einschlußverbindung mit Cyclodextrinen oder Cyclodextrinderivaten vorliegt sowie in Form von Granulaten, Kau- oder Brausetabletten.

Thioctsäure (alpha-Liponsäure) ist chemisch eine 1,2-Dithiacyclopentan-3-valeriansäure. Die Erfindung bezieht sich nicht nur auf die razemische Form, sondern ebenso auf die reine (R)-beziehungsweise (S)-Thioctsäure sowie auf Gemische aus (R)- und (S)-Thioctsäure mit beliebiger Zusammensetzung. Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen alpha-Ketosäuren. Thioctsäure wird seit längerer Zeit bei verschiedenen Erkrankungen eingesetzt, so unter anderem bei Lebererkrankungen, bei Leberschädigungen durch Pilzvergiftung sowie bei diabetischer und alkoholischer Polyneuropathie, einer mit Stoffwechselerkrankungen einhergehenden Veränderung peripherer Nerven. Enantiomerenrein sind (R)- und (S)-Thioctsäure beispielsweise nach den Vorschriften im EP 261 336 zugänglich.

Dihydroliponsäure ist 6,8-Dimercapto-octansäure. Dihydroliponsäure stellt die reduzierte Form der Thioctsäure dar. Aus Tieruntersuchungen ist bekannt, daß Dihydroliponsäure Schlangengift inaktiviert.

Die derzeit handelsüblichen thioctsäurehaltigen Tablettenzubereitungen enthalten maximal 600 mg Thioctsäure in einer Filmtablette zu 850 mg.

Im Anfangsstadium der Behandlung und bei der Behandlung von AIDS werden jedoch hohe Dosierungen von Thioctsäure verabreicht, die bei peroraler Applikation bis zu mehreren Gramm täglich betragen können. Bis zu einer Dosierung von 300 mg Thioctsäure sind Filmtabletten in einer Form herstellbar, die noch einigermaßen schluckbar ist. Bei höheren Dosierungen nehmen die Formlinge jedoch eine Größe an, die sich negativ auf die Patienten-Compliance auswirkt. Derartige Tabletten sind auch im Magen-Darm-Trakt schlecht verträglich und lassen sich somit nicht mehr applizieren.

Erfolglos wurde versucht, das Problem durch die Gabe einer Lösung von Thioctsäure in Form ihrer Salze zu lösen, wie dies bereits in der europäischen Anmeldung 0 427 246 A 2 vorgeschlagen wurde, beispielsweise als Lysin-, Arginin-, Ethylendiamin- oder Trometamolsalz. Diese Thioctsäuresalze weisen aber einen unbefriedigenden Geschmack auf.

Ebenfalls erfolglos wurde versucht, das Problem durch Gabe einer Suspension der freien Thioctsäure zu lösen.

Die Thioctsäure zeichnet sich aber durch einen stark brennenden Geschmack aus, der lange anhalt. Der Weg der Applikation als Trinksuspension ließ sich daher bisher ebenfalls nicht beschreiten.

Trinklösungen haben darüber hinaus den Nachteil, daß sie in angebrochenen Flaschen nicht stabil sind und infolgedessen in Einzeldosisbehälter abgefüllt werden müssen. Zu diesem Zweck wird die Lösung in Glasampullen eingeschweißt. Die Herstellung der Ampullen ist aufwendig, desgleichen die Verpackung. Die Ampulle muß vom Patienten vor der Anwendung erst geöffnet werden. Dies ist für die orale Anwendung ein umständliches Vorgehen. Zudem hat die Lösung ein relativ hohes Gewicht und beansprucht viel Platz für die Lagerung. Der wesentliche Nachteil der Trinklösung ist aber der sehr unangenehme Geschmack, so daß sie trotz Aromatisierung von Probanden sehr ungern eingenommen wird.

Es besteht also ein dringender Bedarf nach Verfahren, die es erlauben, Thioctsäure in hohen Dosierungen mit. guter Patienten-compliance peroral zu applizieren. Dieses Ziel wird erfindungsgemäß dadurch erreicht, daß Thioctsäure nicht in gelöster oder komprimierter Form, sondern als Granulat, oder Kautablette oder in Form einer in Flüssigkeit aufgelösten Brausetablette appliziert wird. Ein Gegenstand der Erfindung ist somit ein Granulat oder eine Kautablette oder eine Brausetablette, enthaltend als Wirksubstanz Thioctsäure oder deren Salze. Das Granulat kann entweder direkt eingenommen, mit Nahrung vermischt oder in Flüssigkeit suspendiert bzw. aufgelöst durch Essen oder Trinken appliziert werden. Die genannten Darreichungsformen haben außerdem den Vorteil der verbesserten Verträglichkeit und Bioverfügbarkeit. Das Granulat kann in Beutel abgefüllt, zu Kautabletten verpreßt oder nach Zufügung einer physiologisch unbedenklichen Brausemischung zu Brausetabletten verpreßt werden.
Es enthält, bezogen auf einen Gewichtstell Thioctsäure
0.001 - 1 Gewichtsteile Bindemittel
0.001 - 0.1 Gewichtstelle Fließhilfsmittel
sowie gegebenenfalls Netzmittel und physiologisch unbedenkliche Geschmacks-, Süß-und/oder Aromastoffe. Im Falle einer Kautablette wird das Granulat mit 0.01 - 0.02 Gewichtstellen eines Gegenklebemittels und gegebenenfalls weiteren Geschmackskorrigentien wie beispielsweise Fructose, Xylit, Sorbit oder Mannit vermischt und zu Tabletten verpreßt.

Im Falle einer Brausetablette wird das Granulat mit 0.05 - 30 Gewichtsteilen einer üblichen physiologisch unbedenklichen Brausemischung vermengt und nach Zufügen von 0.01 -0.02 Gewichtsteilen eines Gleitmittels zu Tabletten verpreßt.

Zur Geschmackskaschierung und Verbesserung der Verträglichkeit empfiehlt es sich, die Thioctsäure nicht in freier Form, sondern in Form ihrer Salze mit physiologisch unbedenklichen Basen oder in Form von Einschlußkomplexen, Einbettungen oder umhüllten Partikeln einzusetzen. Es Ist auch möglich, die Salze der Thioctsäure als Einbettungen oder umhüllte Partikel einzusetzen.

Als Salzbildner der Thioctsäure kommen in Frage: Basische Aminosäuren wie Arginin oder Lysin, physiologisch verträgliche Alkali- oder Erdalkalihydroxide-, -carbonate oder -hydrogencarbonate,Ammoniumhydroxid, Amine der Former N R₁ R₂ R₃ worin die Reste R₁, R₂ und R₃ gleich oder verschieden sind und Wasserstoff, C₁-C₄-Alkyl oder C₁-C₄ Hydroxyalkyl bedeuten wie beispielsweise Mono-und Diethanolamin, 1-Amino-2-propanol, 3-Amino-1-propanol; Alkylendiamine mit einer Alkylenkette aus 2 bis 6 C-Atomen wie Ethylendiamin oder Hexamethylentetramin, gesättigte cyclische Aminoverbindungen mit 4 - 6 Ringkohlenstoffatomen wie Piperidin, Piperaztn, Pyrrolidin, Morpholin; N-Methylglucamin, Kreatin, Trometmol, N-Methyl-morpholin. Bevorzugt werden Salze mit Erdalkalimetallen (Calcium-, Magnesiumsalz), basischen Aminosäuren und Trometamol. Die im folgenden angegebenen Mengen für Thioctsäure sind im Falle des Einsatzes der Salze entsprechend dem Molekulargewicht umzurechnen.

Zur peroralen Applikation hoher Dosierungen der Thioctsäure besteht, wie erwähnt, ein dringender Bedarf nach Verfahren, die es erlauben, den brennenden Geschmack der Thioctsäure abzumildern bzw. ganz zu eliminieren.

Das gleiche Problem liegt vor bei der peroralen Applikation von Dihydroliponsäure. Es besteht jedoch nicht nur ein Bedarf nach Verfahren, die eine verbesserte perorale Appliation der Thioctsäure und Dihydroliponsäure erlauben, sondern auch ein Bedarf nach verbesserten Formen für andere Appilkationsformen. So war die rektale Applikation der Thioctsäure bisher nicht möglich gewesen, da nach der Applikation ein stark brennendes Gefühl im Rektum auftrat. Auch hier besteht der Bedarf nach Formulierungen, die eine rektale Applikation erlauben.

Die inhalative (pulmonale) Applikation der Dihydroliponsäure in Form der wässrigen Lösung eines Salzes war bisher nicht durchführbar, da es bereits bei der Vernebelung der entsprechenden Lösung zu einer Oxydation der Dihydroliponsäure kam. Aus diesem Grund ist auch die Herstellung peroraler Darreichungsformen von Dihydroliponsäure mit guter Langzeitstabilität problematisch.

Enantiomere der Thioctsäure weisen niedrige Schmelzpunkte auf (R-Thioctsäure: 47 °C; S-Thioctsäure: 46 °C). Die Herstellung von schnell zerfallenden Tabletten im industriellen Großmaßstab wird dadurch sehr erschwert, weil es bei der Aufbereitung der Wirkstoffe zur Herstellung der Tabletten (z.B. Trocknen des Granulats, Auftragen und Trocknen des Filmes bei Filmcoating) zur Erwärmung der Tabletten und damit zum Sintern des niedrigschmelzenden Wirkstoffes kommen kann. Dies hat Auswirkungen auf die Zerfallszelt der Tabletten und auf die Bloverfügbarkeit des Wirkstoffes.

Ein weiterer Nachteil der enantiomerenreinen Formen der Thioctsäure ist die Tatsache, daß die reine R- und S-Thioctsäure leicht zur Polymerisation neigen.

Gegenstand der Erfindung ist daher die Bereitstellung von Darreichungsformen der Thioctsäure (R-Enantiomer oder S-Enantiomer oder Racemat) sowie Dihydroliponsäure (auch hier sowohl R-Enantiomer oder S-Enantiomer oder Racemat) mit der Möglichkeit der peroralen Applikation hoher Dosen mit besserem Geschmack, sowie der Möglichkeit der lokalen (rektalen, pulmonalen, nasalen, dermalen, vaginalen, ophthalmischen) Applikation sowie der vereinfachten Herstellung von peroralen Darreichungsformen der R-bzw. S-Thioctsäure sowie Dihydroliponsäure mit verbesserter Bloverfügbarkeit.

Die gleichzeitige Lösung der oben geschilderten Probleme wird überraschenderweise dadurch erreicht, daß zur Herstellung der Darreichungsformen Thioctsäure oder Dihydroliponsäure in Form ihrer Einschlußverbindungen mit Cyclodextrinen oder Cyclodextrinderivaten eingesetzt werden. Geeignet hierzu sind alle Cyclodextrine bzw. Cyclodextrinderivate., die zur Bildung eines Elnschlußkomplexes mit Thioctsäure bzw. Dihydroliponsäure befähigt sind.

Es wurde nun gefunden, daß die Einschlußverbindung aus R-Thioctsäure und Cyclodextrinen, beispielsweise β-Cyclodextrin stabil ist. Ebenso ist die Einschlußverbindung aus S-Thioctsäure und Cyclodextrinen, beispielsweise β-Cyclodextrin stabil.

Die Verwendung von Cyclodextrinen zur Herstellung von Elnschlußverbindungen ist im allgemeinen bereits bekannt. Eine Reihe von Patentanmeldungen sowie Publikationen schildert mögliche Einsatzgebiete. Insbesondere die Verwendung von Cyclodextrinderivaten war in der Vergangenheit Gegenstand von Untersuchungen (siehe hierzu Europäische Patentanmeldungen 0 312 352, 0 381 747, 0 463 653, 0 512 050, 0 149 197).

EP 427 247 offenbart als Komplexbildner für R- oder S-α-Liponsäure Harnstoff, Thioharnstoff, Cyclodextrine und Amylose.

Diese Komplexbildner werden im Rahmen der üblichen pharmazeutischen Herstellungsmethodik eingesetzt. Irgendwelche vorteilhafte Effekte sind mit dem Einsatz der Komplexbildner nicht verbunden. Gegebenenfalls kann der Zusatz weiterer Stabilisatoren, beispielsweise Puffer, erforderlich sein.

Bei den erfindungsgemäßen Verbindungen aus R-Thioctsäure und β-Cyclodextrin und aus S-Thioctsäure und β-Cylodextrin kann auf den Einsatz weiterer Stabillsatoren verzichtet werden.

Bisherige Publikationen über den möglichen Einsatz von Cyclodextrinen beschäftigen sich überwiegend mit der Verbesserung der Löslichkeit von schwerlöslichen Substanzen oder mit der Verbesserung des transdermalen oder nasalen Transports von Wirkstoffen.

In keiner dieser Patentanmeldungen oder Publikationen sind Elnschlußverbindungen aus Cyclodextrinen oder Cyclodextrinderivaten mit Thioctsäure oder Dihydroliponsäure beschrieben, insbesondere nicht die Vorteile, die mit der Verwendung der Einschlußverbindungen im Vergleich zur freien Thioctsäure oder Dihydroliponsäure verbunden sind.

So war zwar die Verbesserung des Geschmacks durch Herstellung von Einschlußverbindungen von schlecht schmeckenden Substanzen auch bereits beschrieben. Tatsache ist aber, daß es viele Wirksubstanzen gibt, bei denen die Verbesserung des Geschmacks völlig unzureichend ist (beispielsweise Azelastin). Es ist überraschend, daß sich im Falle der Thioctsäure und Dihydroliponsäure der stark brennende Geschmack durch. Einbettung in Cyclodextrine völlig inhibieren läßt. Gleichzeitig ist dabei aber die Bloverfügbarkeit dieser Substanzen im Vergleich zu den freien Säuren nicht beeinträchtigt, im Gegenteil sogar erhöht.

Als Cyclodextrine sind einsetzbar: α-Cyclodextrin (Cyclohexaamylose, ringförmig aufgebaut aus 6-Glucose-Einhelten), β-Cyclodextrin (Cycloheptaamylose, ringförmig aufgebaut aus 7-Glucose-Einheiten), γ-Cyclodextrin (Cyclooctaamylose, ringförmig aus 8 Glucose-Einheiten).

Folgende Derivate von α-, β- oder γ-Cyclodextrinen (im folgenden mit CD bezeichnet) sind einsetzbar:

### Methylierte CD; Beispiele:

2.6-Dimethyl-β-CD
Methyl-γ-CD
   Carboxymethyl-CD
   Hydroxyalkyl-CD
      - Beispiele:: Hydroxypropyl-β-CD
      Hydroxyethyl-β-CD
   Ethylierte CD
   Dialkylaminoethylierte CD
   Sulfoalkyl-CD
   Partiell methylierte Carboxyacyl-CD
   Verzweigtkettige CD
      Glucosyl, Diglucosyl, Maltosyl, Dimaltosyl CD
   Dimerisierte CD
      (Dimerisierung beispielsweise über Diester, Diamine oder Diamide).
   Polymerisierte CD

Insbesondere eignet sich das β-Cyclodextrin. Dies nicht zuletzt dadurch, weil es beispielsweise von japanischen Behörden bereits als Lebensmittelzusatzstoff akzeptiert und auch preiswert zu beziehen ist (Hersteller beispielsweise: Wacker Chemie GmbH, München). Für flüssige Zubereitungen eignen sich Insbesondere Hydroxypropyl-β-Cyclodextrin und Methyl-γ-Cyclodextrin.

Die Herstellung des Einschlußkomplexes aus Thioctsäure oder Dihydroliponsäure kann auf verschiedene Weise erfolgen. Am einfachsten ist folgende Methode:

Thioctsäure oder Dihydroliponsäure wird mit der 4,7-bis 50-fachen, bevorzugt der 6- bis 20-fachen, insbesondere der 7- bis 12-fachen Menge (jeweils wasserfrei sowie Gewichtsmengen) Cyclodextrin oder Cyclodextrinderivat in Wasser von etwa 40 °C suspendiert und über mehrere Stunden gerührt. Die Thioctsäure oder Dihydroliponsäure geht dabei in Lösung. Durch Abkühlen fällt die Einschlußverbindung. aus und kann abfiltriert werden.

Nach dem oben geschilderten Verfahren kann sowohl R-als auch S-Thioctsäure in enantiomerenreiner Form eingeschlossen werden.

Bei Einsatz von gut wasserlöslichen Cyclodextrinen oder Cyclodextrinderivaten kann auch so vorgegangen werden, daß nach Auflösung der Thioctsäure oder Dihydroliponsäure die entstandene Lösung direkt weiterverarbeitet wird, beispielsweise zu Emulsionen, Injektionslösungen oder Augentropfen oder die Einschlußverbindung durch Gefrier- oder Sprühtrocknung gewonnen wird.

Eine andere Möglichkeit der Herstellung besteht darin, Thioctsäure oder Dihydroliponsäure zusammen mit cyclodextrin oder Cyclodextrinderivat und gleichen Anteilen Wasser (bezogen auf Cyclodextrin) mehrere Stunden bei 40 °C zu kneten und anschließend die erhaltene Masse zu trocknen.

Die Einschlußverbindungen der Thioctsäure oder Dihydroliponsäure eignen sich zur Herstellung von Granulaten, Pulvern, Tabletten, Kautabletten, Brausetabletten, Brausegranulaten, Pellets, Kapseln, Suppositorien, Cremes, Salben, Lotionen, Emulsionen, Suspensionen, Lösungen. Die Applikation kann erfolgen peroral, Intraarteriell, intrakardial, intravenös, intramuskulär, dermal, rektal, ophthalmisch, nasal, vaginal, pulmonal.

Gegenüber den bisher verfügbaren Zubereitungen sind die Zubereitungen, die die erfindungsgemäßen Einschlußverbindungen enthalten, vorteilhaft nicht nur wegen ihres besseren Geschmacks, sondern auch wegen ihrer besseren Stabilität, Verträglichkeit, Bioverfügbarkeit und vereinfachten Herstellung.

Die Herstellung der erwähnten Darreichungsformen erfolgt nach üblichen in der pharmazeutischen Technologie bekannten Verfahren, jeweils unter Einsatz der erfindungsgemäßen Einschlußverbindung.

Bei der Herstellung von Granulaten wird folgendermaßen gearbeitet:

Beispielsweise wird die getrocknete Einschlußverbindung mit Bindemitteln und Netzmitteln in üblicher Weise granuliert, mit Geschmacks- und/oder Süßungsmitteln gemischt und in Sachets (Beutel) in entsprechender Dosierung abgefüllt. Zur Anwendung wird der Beutelinhalt in Wasser oder Fruchtsaft eingerührt und getrunken. Auf diese Weise lassen sich beliebig hohe Dosierungen bequem peroral applizieren.

Zur Herstellung einer Brausetablette oder eines Brausegranulats wird die Einschlußverbindung in üblicher Weise (beispielsweise im Vakuumgranulator) mit einem Carbonat oder einer Säurekomponente granuliert und die erhaltene Mischung nach Zufügung von Geschmacksstoffen, der noch fehlenden Säurekomponente oder des Carbonats sowie weiteren üblichen Fließregulierungs- und Formtrennmitteln zu Tabletten verpreßt oder in Beutel abgefüllt. Die Anwendung erfolgt ebenfalls durch Eingabe der Tablette oder des Granulats in Wasser.

Die resultierende Suspension ist von angenehmem Geschmack d.h. ohne den bisher üblichen brennenden und schwefelwasserstoffartigen Geschmack.

Eine weitere Methode zur Verbesserung des Geschmacks und der Verträglichkeit besteht In der Einbettung von Thioctäure oder Salzen der Thioctsäure in folgende Substanzen oder Mischungen dieser Substanzen:
- Verdauliche Fette, z.B. Triglyceride von gesättigten Fettsäuren C₈H₁₆O₂ bis C₁₈H₃₆O₂ und deren Gemische,
- hydriertes Erdnußöl, hydriertes Ricinusöl, hydriertes Baumwollsamenöl,Gemische von Mono-, Di-, Triestern der Palmitin- und Stearinsäure mit Glycerin, Glyerintrioleat, Diglykolstearat, Stearinsäure.
- Unverdauliche Fette bzw. fettähnliche Substanzen, z.B. Ester von aliphatischen gesättigten oder ungesättigten Fettsäuren (2 bis 22 C- Atome, insbesondere 10 bis 18 C-Atome) mit einwertigen aliphatischen Alkoholen (1 bis 20 C-Atome). Carnaubawachs, Bienenwachs, Paraffinwachs, Fettalkohole (geradkettig oder verzweigtkettig) der Kettenlänge C₈H₁₇OH bis C₃₀H₆₁OH,, insbesondere C₁₂H₂₅OH bis C₂₄H₄₉OH.
- Polymere wie Polyvinylalkohol, Polyvinylchlorid, Polyacrylsäure (Carbopol®); anionische Polymerisate aus Methacrylsäure und Methacrylsäureestern (Eudragit®L, Eudragit®S), Acryl- und Methacrylsäureester-Copolymerisate mit Trimethylammoniummethacrylat (Eudragit®RL, Eudragit®RS), Copolymerisat von Acrylsäureethylund Methacrylsäuremethylestern (Eudragit®NE 30 D), sowie aus Acrylsäure, Methacrylsäure sowie deren Estern (Verhältnis der freien Carboxylgruppen zu den Estergruppen 1:1) (Eudragit®-L 30 D), Polyethylen, Polyglycolsäure, Polyhydroxybuttersäure, Polymilchsäure, Copolymere aus Milchsäure und Glycolsäure (Hersteller:Boehringer Ingelheim), Copolymere aus Milchsäure und Ethylenoxid, Copolymere aus Glycolsäure und Ethylenoxid, Copolymere aus Milchsäure und Hydroxybuttersäure, Hydroxypropylmethylcellulose-phthalat oder acetatsuccinat; Celluloseacetatphthalat, Stärkeacetatphthalat, sowie Polyvinylacetatphthalat; Methylcellulosephthalat, -succinat, -phthalatsuccinat, Methylcellulose-phthalsäurehalbester; Zein; Ethylcellulose; Schellack, Gluten; Ethylcarboxyethylcellulose; Ethacrylat-Maleinsäureanhydrid-Copolymer; Maleinsäureanhydrid-Vinylmethylether-Copolymer; Styrol -Maleinsäure-Copolymerisate; 2-Ethylhexyl-acrylat-maleinsäureanhydrid; Crotonsäure-Vinylacetat-Copolymer; Glutaminsäure/Glutaminsöureester-Copolymer; Carboxymethylcellulose-glycerinmonooctanoat; Celluloseacetatsuccinat; Polyarginin; quervernetztes Alginat; quervernetzte Gelatine;
- Quellstoffe wie Methylcellulose, Hydroxypropylcellulose, Hydroxypropylmethylcellulose (Pharmacoat), Propylenglykoläther der Methylcellulose (Methocel E), Alginsäure und ihre Salze (Na-,Ca-Salz, auch Mischungen aus Natriumalginat und Calciumsalzen z.B. CaHPO₄), Stärke, Carboxymethylstärke, Carboxymethylcellulose und deren Salze (z.B. Na-Salz), Galaktomannan, Gummi arabicum, Karaya Gummi, Ghatti Gum, Agar-agar, Carrageen, Gelatine, Xanthan-Gummi, Guar Gummi und seine Derivate, Johannisbrotkernmehl, Propylenglykolalginat, Pektin, Traganth, Gum Acacia.

Auf 1 Gewichtsteil Thioctsäure werden bei diesen Einbettungsmaterialien 0.1 - 20 Gewichtstelle Einbettungsmaterial, vorzugsweise 0.15 - 15, insbesondere 1 bis 10 Gewichtsteile Einbettungsmaterial eingesetzt.

Die Herstellung dieser Einbettungen erfolgt bei Temperaturen zwischen 18 C und 80 C. Die Herstellung kann erfolgen
a) durch Lösen oder Dispergieren von Thioctsäure oder ihren Salzen in den genannten Fetten oder fettähnlichen Substanzen oder Mischungen davon, auch unter Schmelzen der genannten Substanzen und anschließend Wiederabkühlen, Zerkleinern. Das Abkühlen der Schmelze und Zerkleinern kann auch in einem Schritt zusammengefaßt werden, indem die Schmelze in kaltem Wasser dispergiert wird oder einer Sprüherstarrung unterworden wird.
b) durch Mischen von Thioctsäure oder ihren Salzen mit den genannten Fetten, Polymeren oder Quellstoffen oder Mischungen dieser Substanzen, auch unter Anwendung von Wärme und Zerkleinern
c) durch Mischen von Thioctsäure oder ihren Salzen mit Lösungen der genannten Fette oder Polymeren in Wasser oder organischen Lösungsmitteln, wie z.B. Ethylacetat, Aceton oder Isopropanol, evtl. Mischen mit Trägermaterialien wie Cellulosen, sowie nachfolgendes Abdampfen der Lösungsmittel oder Sprühtrocknung und Vermischen der erhaltenen Wirkstoffeinbettung mit weiteren Hilfsstoffen
d) durch Anfeuchten einer Mischung aus Thioctsäure oder ihren Salzen und den genannten Quellstoffen mit organischen Lösungsmitteln wie Ethanol, Ethylacetat, Aceton oder Isopropanol, evtl. unter Beifügung von Bindemitteln wie Polyvinylpyrrolidon oder Copolymeren aus Polyvinylpyrrolidon und Polyvinylacetat. Granulieren der erhaltenen Mischung, nachfolgendes Trocknen.

Eine Verbesserung von Geschmack und Verträglichkeit kann auch erfolgen durch Umhüllung von Granulat, das Thioctsäure oder Salze der Thioctsäure mit physiologisch verträglichen Salzbildnern enthält. Hierbei können Überzüge verwendet werden, die sich beispielsweise erst im Magensaft oder Intestinaltrakt auflösen. Zur Herstellung der Überzüge können beispielsweise verwendet werden: Copolymerisate von Dimethylaminoacrylsäure und neutralen Methacrylsäureestern, die in Wasser und Speichel unlöslich sind, im sauren Bereich aber löslich (zum Beispiel Eudragt®E; Hersteller: Röhm GmbH). Man kann aber auch magensaftresistente Lacke einsetzen wie zum Beispiel Hydroxypropylmethylcellulosephthalat oder -acetatsuccinat; Stärke-, sowie Polyvinylacetatphthalat; Carboxymethylcellulose; Polyvinylacetat; Methylcellulose-phthalat, Methylcellulose-succinat, Methylcellulose phthalatsuccinat sowie Methylcellulose-phthalsäurehalbester; Zein, Ethylcellulose sowie Ethylcellulose-succinat; Schellack; Gluten; Ethylcarboxyethylcellulose; Ethacrylat-Maleinsäureanhydrid-Copolymer; Maleinsäureanhydrid-Vinylmethylether-Copolymer; Styrol -Maleinsäure-Copolymerisate; 2-Ethylhexyl -acrylat-maleinsäureanhydrid; Crotonsäure-Vinylacetat- Copolymer; Glutaminsäure/Glutaminsäureester-Copolymer; Carboxymethylcelluloseglycerinmonooctanoat; Celluloseacetatsuccinat; Polyarginin; Fette, Wachse, Fettalkohole;Anionische Polymerisate aus Methacrylsäure und Methacrylsäureestern (Eudragit®L, Eudragit®S); Copolymerisate aus Acryl- und Methacrylsäureestern mit einem geringen Gehalt an Ammoniumgruppen (Eudragit®RS), sowie Copolymere aus Acryl- und Methacrylsäureestern und Trimethylammoniummethacrylat (Eudragit®RL), Copolymerisat aus Acrylsäureäthyl- und Methacrylsäure-methylestern 70:30 (Eudragit®NE 30 D), Copolymerisat aus Acrylsäure, Methacrylsäure sowie deren Estern (Verhältnis der freien Carboxylgruppen zu den Estergruppen z.B.: 1:1) (Eudragit®L 30 D).

Die genannten Substanzen können zusätzlich übliche Weichmacher (z.B. Dlbutylsebacat, Citronen- und Weinsäureester, Glycerin und Glycerinester, Phthalsäureester und ähnliche Stoffe) enthalten, außerdem den Zusatz wasserlöslicher Substanzen wie Polyethylenglykole, Polyvinylpyrrolidon, Copolymerisat aus Polyvinylpyrrolidon und Polyvinylacetat, Hydroxypropylcellulose, Hydroxypropylmethylcellulose. Auch der Zusatz vom Feststoffen wie Talkum und/oder Magnesiumstearat in der Umhüllung ist möglich.

Die Umhüllung erfolgt durch Aufsprühen von Lösungen in organischen Lösungsmitteln oder Wasser, wobei noch weitere Hilfsstoffe zur Optimierung deren Verarbeitbarkeit zugesetzt sein können, wie z.B. oberflächenaktive Substanzen, Pigmente.

Das Aufsprühen erfolgt z.B. im Dragierkessel oder in perforierten Kesseln, oder im Luftsuspensionsverfahren (z.B. Glatt Wirbelschichtanlage WLSD 5).

Die Umhüllung kann auch im Koazervationsverfahren erfolgen, wobei sogenannte Mikrokapseln gebildet werden. Die Umhüllung kann auch durch Koagulation wässriger Dispersionen der zuvor genannten Substanzen erfolgen, indem der Wirkstoff mit der Dispersion vermischt und das Wasser durch Trocknen entfernt wird.

Auf das Granulat sollen 0.0125 - 2 Gewichtsteile Lacktrockensubstanz, bezogen auf ein Gewichtsteil Thioctsäure aufgetragen werden. Darüber hinaus ist es möglich, das Thioctsäure-Granulat mit Xanthan und/oder Maltodextrin und Fumarsäure zu umhüllen, wie in DE 34 40 288 beschrieben. Als Granulatpartikel gelten unregelmäßig geformte oder regelmäßig geformte (beispielsweise sphärische oder zylindrische) Körper, die einen Durchmesser von 0,05 bis 3 mm aufweisen. Eine weitere Möglichkeit besteht in der Umhüllung von Thioctsäure oder deren Salzen mit Cyclodextrinen oder Cyclodextrinderivaten. Dabei ist überraschend, daß trotz der gegenüber einer echten Wirkstoffeinbettung geringen Menge an Cyclodextrin eine Maskierung des brennenden Geschmacks möglich ist. Offenbar reicht eine oberflächliche Ausbildung von Einschlußkomplexen für die geschmackliche Maskierung der Thioctsäure aus. Beispielsweise reicht es aus, Thioctsäure mit 1 bis 3 Gewichtsteilen Cyclodextrin bezogen auf 1 Gewichtsteil Thioctsäure zu umhüllen. Besonders geeignet ist wieder das β-Cyclodextrin.

Die bisher beschriebenen Einbettungen von Thioctsäure und Dihydroliponsäure sowie deren Salze in Cydodextrine, verschiedene Einbettungssubstanzen oder in Form umhüllter Partikel können als Kau- oder Brausetablette oder als Granulat (auch Brausegranulat) formuliert vorliegen. Falls es sich bei der erfindungsgemäßen Form um ein Brausegranulat oder eine Brausetablette handelt, enthält diese zusätzlich eine übliche physiologisch unbedenkliche Brausemischung. Die Menge dieser Brausemischung, bezogen auf einen Gewichtsteil Thioctsäure, beträgt 0,05 bis 30 Gewichtstelle. Eine solche besteht aus einer Substanz, die in Gegenwart einer Säure in wässrigem beziehungsweise alkoholischem Milieu Kohlendioxid entwickelt (CO₂-Lieferer) und einer physiologisch verträglichen organischen Säure.

Als CO₂-liefernde Substanzen kommen beispielsweise in Frage: Carbonate oder Hydrogencarbonate des Natriums, Kaliums, Magnesiums oder Calciums, im Falle von Magnesium und Calcium vorzugsweise die neutralen Carbonate. Auch Mischungen dieser Carbonate sind möglich. Als organische Säuren, die aus solchen Carbonaten Kohlendioxid freisetzen, kommen beispielsweise in Frage: organische gesättigte oder ungesättigte Di- und Tricarbonsäuren mit 2 bis 8, vorzugsweise 2 bis 6 C-Atomen, die auch eine, zwei, drei oder vier, vorzugsweise eine oder zwei Hydroxygruppen enthalten können. Beispiele hierfür sind: Citronensäure, Weinsäure, Adipinsäure, Maleinsäure, Fumarsäure. Ebenfalls ist Alginsäure verwendbar. Auch Gemische der genannten Säuren sind möglich.
Auf einen Gewichtsteil Thioctsäure werden beispielsweise 0.02 - 16 Gewichtsteile der CO₂ -liefernden Komponente der Brausemischung und 0.03-12 Gewichtsteile der zugehörigen Säurekomponente verwendet. Bei der Herstellung der Brausetabletten kann zum Beispiel die komplette Brausemischung als solche nach der Granulierungsstufe zugesetzt werden. Es ist aber auch möglich beziehungsweise bevorzugt, die Komponente der Brausemischung, die das Kohlendioxid liefert, vor der Granulierung zuzusetzen und die entsprechende zugehörige Säurekomponente dann später dem trockenen Granulat zusammen mit beispielsweise dem Gleitmittel, den Geschmack-, Süß-und Aroma-stoffen sowie gegebenenfalls den restlichen Füll-, Binde- und Sprengmitteln beizumischen.

Zur Herstellung des Granulates oder der Kau-oder Brausetabletten werden übliche Verfahren angewendet, wie sie In der pharmazeutisch-technologischen Literatur beschrieben sind (beispielsweise in dem Standardwerk Sucker, Fuchs, Speiser, Pharmazeutische Technologie, Thieme Verlag, Stuttgart). Die Herstellung (alle Schritte außer Trocknung) erfolgt zum Beispiel bei Temperaturen zwischen 10 C und 80 C, vorzugsweise 18 C bis 50 C insbesondere 20 C bis 30 C. Die Trocknung des Granulats erfolgt beispielsweise zwischen 30 C und 80 C vorzugsweise 40 C bis 70 C. Bei der Herstellung können alle pharmazeutisch gebräuchlichen Bindemittel wie Cellulosederivate (zum Beispiel Ethylcellulose, Hydroxyethylcellulose, Carboxymethylcellulose, Methylcellulose, Hydroxypropylmethylcellulose), Gelatine, Stärke, Polyglycole (mittlere Molmasse 1000 - 3500 Dalton), Polyvinylalkohole, Polyvinylpyrrolidon, Polyacrylsäure, Vinylpyrrolidon-VinylacetatCopolymerisat, Alginate, Saccharose oder Glucose, Polysaccharide, wie zum Beispiel natürliche Gummisorten, wie zum Beispiel Gummi Arabicum, Tragant, Pektin, Cyclodextrin, Guar-Gummi in Mengen von 1 -30 Gewichts%, vorzugsweise 5 - 20 Gewichts%, insbesondere 10 - 15 Gewichts% bezogen auf die Granulatmenge eingesetzt werden (Konzentration der wässrigen Bindemittellösungen 2 -30 Gewichts% vorzugsweise 5 - 15 % Gewichts%). Dabei können auch gleichzeitig verschiedene Bindemittel, zum Beispiel unterschiedliche Cellulosederivate nebeneinander, eingesetzt werden. Die Bindemittel können in die trockene Pulvermischung eingearbeitet werden oder in der Granulierflüssigkeit gelöst oder dispergiert eingebracht werden.

Auch die Kombination von trocken vorgelegtem und gelöstem beziehungsweise dispergiertem Bindemittel ist geeignet. Falls erforderlich, wird das Granulat mit Füll-, Binde-, Spreng-, Netz-, Fließhilfsmittel, Schmiermittel und/oder Gegenklebemittel versetzt.

Als Füllmittel können beispielsweise eingesetzt werden: Cellulose, Cellulosederivate, Saccharose, Lactose, Glucose, Fructose, Calciumphosphate, Calciumsulfate, Calciumcarbonate, Stärke, modifizierte Stärke, Zuckeralkohole wie Sorbitol oder Mannitol.

Als Sprengmittel können beispielsweise eingesetzt werden: Stärke, modifizierte Stärke (beispielsweise Natrium-Stärkeglycolat, Starch 1500), Cellulose, Cellulosederivate, Alginate, quervernetztes Polyvinylpyrrolidon oder quervernetzte Carboxymethylcellulose (Ac-Di-Sol/FMC).

Zur Verbesserung des Suspendierung in Flüssigkeiten kann das Granulat oder die Brausetablette Netzmittel enthalten. Als Beispiele seien genannt: Alkaliseifen, wie Alkalisalze höherer Fettsäuren (z.B. Na-palmitat, Na-stearat oder deren Derivate (z.B. Narizinolatschwefelsäureester); sulfurierte Verbindungen oder sulfonierte Verbindungen, die durch Umsetzung von höheren Fettalkoholen mit Schwefelsäure oder Chlorsulfonsäure entstehen und z.B. als Natriumsalze eingesetzt werden (z.B. Natriumlaurylsulfat, Natriumcetylsulfat, Natriumstearylsulfat, Natriumcetylsulfonat); Salze der Gallensäuren; Saponine; quartäre Ammoniumverbindungen; Partialfettsäureester des Sorbitans; Partialfettsäureester und Fettsäureester des Polyoxyethylensorbitans; Sorbitolether des Polyoxyethylens; Fettalkoholether des Polyoxyethylens; Fettsäureester der Saccharose; Fettsäureester des Polyglycerols; Proteine; Lecithine. Als Fileßhilfsmittel eignen sich beispielsweise: Kolloldales Siliciumdioxid, Talkum oder Magnesiumstearat. Als Schmiermittel können beispielsweise eingesetzt werden: Magnesiumstearat, Calciumstearat, D,L-Leucin, Talkum, Stearinsäure, Polyglycole (mittlere Molmasse 3000 - 35000), Fettalkohole oder Wachse. Als Gegenklebemittel sind beispielsweise einsetzbar: Stärke, Talkum, Magbesiumstearat, Calciumstearat oder D,L-Leucin.

Falls das Granulat als solches als Arzneimittel verwendet wird, beispielsweise als Trinkgranulat, enthält dieses vorzugsweise neben dem Wirkstoff Thioctsäure oder Dihydroliponsäure oder deren Salze nur ein Bindemittel. Bei dem Bindemittel kann es sich um eines der angegebenen Bindemittel handeln, aber es können auch Gemische solcher Bindemittel verwendet werden. Vorzugsweise verwendet man 0.01 - 1 Gewichtsteile Bindemittel auf einen Gewichtsteil Thioctsäure. Die obere Korngröße eines solchen Granulats liegt bei 3 mm.

Das Granulat, die Kau- oder Brausetablette enthalten beispielsweise 100 mg bis maximal 3 g Thioctsäure. Thioctsäure ist im allgemeinen in einer solchen Menge vorhanden, daß es 10 - 80 Gewichtsprozent ausmacht. Es empfiehlt sich darüberhinaus, Geschmack-, Süß- und/oder Aromastoffe zuzumischen. Als Aromastoffe kommen zum Beispiel folgende Pulveraromen in Frage: Ananas, Apfel, Aprikose, Himbeere, Kirsche, Kola, Orange, Passionsfrucht, Zitrone Grapefruit, Vanille, Schokolade. Das Granulat soll 0.05 - 0.2 Gewichtsteile Aroma, bezogen auf 1 Gewichtsteil Thioctsäure enthalten.

Als Sußungsmittel können folgende Stoffe eingesetzt werden: Saccharin und sein Natriumsalz, Cyclaminsäure und ihr Natriumsalz, Ammoniumglycyrrhizinat, Fructose, Xylit, Sorbit, Mannit, Aspartam, Acesulfam-K. Besonders bevorzugt ist eine Mischung aus 1 Teil Saccharin-Natrium und 10 Teilen Natriumcyclamat (jeweils Gewichtsteile). Das Granulat soll 0.003-12 Gewichtsteile Sußungsmittel, bezogen auf 1 Gewichtsteil Thioctsäure oder Dihydroliponsäure enthalten.

### Beispiel 1:

### Trinkgranulat mit 1000 mg Thioctsäure als Einschlußverbindung mit β-Cyclodextrin

136,2 g β-Cyclodextrin werden in Wasser bei 40 C gelöst. Nach Zugabe von 12 g fein gepulverter Thioctsäure wird bei 40 C weiter gerührt, bis die Thioctsäure in Lösung gegangen ist. Reste von ungelöster Thioctsäure werden abfiltriert. Das Filtrat wird auf +5 C abgekühlt und nach 12 Stunden das gebildete Präzipitat abfiltriert. Es wird bis zur Gewichtskonstanz bei 40 C getrocknet. Die Ausbeute beträgt 68 g (= 85 %). Die Einschlußverbindung enthält etwa 15 % Thioctsäure. Die Einschlußverbindung mit β-Cyclodextrinen kann auch hergestellt werden, indem 1261 g β-Cyclodextrin (Wassergehalt etwa 10%) mit 1250 g Wasser vermischt und die Mischung auf 40°C erwärmt wird. Anschließend werden 206,3 g Thioctsäure zugesetzt und 4 Stunden bei 40°C geknetet. Danach wird die Masse bei 30°C im Vakuum bei 3 mbar getrocknet und gesiebt.

50 g der Einschlußverbindung werden mit 1 g Copolyvidon und 0,5 g Saccharin-Natrium gemischt und durch Zusatz von wenig gereinigtem Wasser in üblicher Weise granuliert. Das getrocknete, gesiebte Granulat wird mit 2 g Geschmackskorrigens und 0,5 g Hochdispersem Siliciumdioxid gemischt und zu 7,2 g in Beutel abgefüllt. Ein Beutel enthält 1000 mg Thioctsäure als Einschlußverbindung mit β-Cyclodextrin. Statt dem Racemat der Thioctsäure kann auch das R-Enantiomer oder das S-Enantiomer eingesetzt werden.

### Beispiel 2:

### Trinkgranulat mit 1000 mg Dihydroliponsäure als Einschlußverbingdung mit β-Cyclodextrin

Es wird gearbeitet wie in Beispiel 1, jedoch wird statt Thioctsäure Dihydroliponsäure eingesetzt. Ein Beutel enthält 1000 mg Dihydroliponsäure als Einschlußverbindung mit β-Cyclodextrin.

### Beispiel 3:

### Kautablette mit 200 mg Dihydroliponsäure oder R-Thioctsäure als Einschlußverbindung mit β-Cyclodextrin

200 g der entsprechend Beispiel 2 hergestellten Einschlußverbindung Dihydroliponsäure / β-Cyclodextrin werden mit 50 g Sorbit und 0,50 g Saccharin-Natrium gemischt und durch Zusatz von Wasser in üblicher Weise granuliert. Nach dem Trocknen und Sieben werden 10 g Talkum sowie eine Verreibung aus 1 g Pfefferminzöl in 2 g Hochdispersem Siliciumdioxid zugesetzt und die erhaltene Mischung zu Tabletten vom Gewicht 1,76 g verpreßt. Statt Dihydroliponsäure kann auch R-Thioctsäure eingesetzt werden.

### Beispiel 4:

Suppositorium mit 50 mg Thioctsäure als Einschlußverbindung mit β-Cyclodextrin 1,78 kg Hartfett*) werden geschmolzen unddarin 20g Sojalecithin eingearbeitet. Anschließend werden in die Schmelze 500 g der entsprechend Beispiel 1 hergestellten Einschlußverbindung Thioctsäure/β-Cyclodextrin eingerührt und intensiv homogenisiert. Die erhaltene Suspension wird zu 2.3 g in Hohlzellen ausgegossen und abgekühlt.
*) Hartfett ist ein Gemisch von Mono-, Di- und Triglyceriden der gesättigten Fettsäuren C₁₀H₂₀O₂ bis C₁₈H₃₆O₂

### Beispiel 5:

### Augentropfen mit 1 % Thioctsäure als Einschlußverbindungen mit Hydroxypropyl-β-Cyclodextrin

120 g Hydroxypropyl-β-Cyclodextrin (Hersteller: Wacker Chemie GmbH, München) werden in 750 ml Wasser für Injektionen gelöst. Bei einer Temperatur von 40 C werden 10 g Thioctsäure zugegeben und bis zur Lösung gerührt. Die Lösung wird mit Wasser für Injektionen auf 1000 ml aufgefüllt, nach sorgfältiger Durchmischung durch ein Membranfilter der Porenweite 0,2 µm steril filtriert und unter aseptischen Bedingungen zu 0,4 ml in Eindosenbehälter abgefüllt. Statt Thioctsäure kann auch Dihydroliponsäure eingesetzt werden.

### Beispiel 6:

### Kautablette mit 400 mg Thioctsäure als Einschlußverbindung mit β-Cyclodextrin

200 g der entsprechend Beispiel 1 hergestellten Einschlußverbindung Thioctsäure/β-Cyclodextrin werden mit 50 g Sorbit und 0.50 g Saccharin-Natrium gemischt und durch Zusatz von Wasser in üblicher Weise granuliert. Nach dem Trocknen und Sieben werden 10 g Talkum sowie eine Verreibung aus 1 g Pfefferminzöl in 2 g Hochdispersem Siliciumdioxid zugesetzt und die erhaltene Mischung zu Tabletten vom Gewicht 3.52 g verpreßt.
Statt dem Racemat der Thioctsäure kann auch das R-Enantiomer oder S-Enantiomer eingesetzt werden.

### Beispiel 7

### Granulat mit 1000 mg Thioctsäure als Einschlußverbindung mit α-Cyclodextrin

33,01 g Thioctsäure werden durch ein Sieb der Maschenweite 0,5 mm gesiebt. 172,8 g α-Cyclodextrin (enthält ca. 10 % Wasser) werden in 700 g Wasser bei 50°C suspendiert. Dazu werden 3,3 g Na-Ascorbat gegeben. Anschließend wird die gesiebte Thioctsäure dazugegeben. Die so erhaltene Suspension wird bei 50°C 3 Stunden gerührt. Anschließend wird die Suspension filtriert und das Filtrat abgekühlt (5°C, 12 Stunden).
Der dabei entstandene Niederschlag wird durch Filtration abgetrennt und im Vakuumtrockenschrank getrocknet (30°C, 2 Stunden, Druck < 10 mbar). Das so hergestellte Pulver enthält 157,8 mg Thioctsäure pro Gramm Pulver. 50 g dieser Einschlußverbindung werden mit 1 g Copolyvidon und 0,5 g Saccharin-Natrium gemischt und durch Zusatz von gereinigtem Wasser in üblicher Weise granuliert. Das getrocknete Granulat wird mit 2 g Geschmackskorrigens und 0,5 g Hochdispersem Siliciumdioxid gemischt und zu 6,84 g in Beutel abgefüllt. Ein Beutel enthält 1000 mg Thioctsäure als Einschlußverbindung mit α-Cyclodextrin.

### Beispiel 8

### Trinktablette mit 600 mg Thioctsäure

In 3 l gereinigtem Wasser werden 60 g Natriumalginat gelöst, 300 g Thioctsäure eingerührt und homogenisiert. Diese Suspension wird mit Hilfe eines Wirbelschichtsprühgranulats auf eine Mischung, bestehend aus 40 g Hochdispersem Siliciumdioxid und 250 g vernetztem Polyvidon, gesprüht. Das so entstandene Granulat wird gesiebt und erneut im Wirbelschichtsprüchgranulator mit einer Lösung, bestehend aus 96 g Calciumchlorid in 900 ml gereinigtem Wasser, besprüht.
Ds so entstandene Produkt wird gesiebt, mit 5 g Magnesiumstearat und 4 g Hochdispersem Siliciumdioxid vermischt und zu Tabletten vom Gewicht 1510 mg verpreßt. Eine Tablette enthält 600 mg Thioctsäure. Die Tablette wird zur Anwendung in Wasser zerfallen gelassen.

## Patentansprüche

1. Einschlußverbindung aus (R)-Thioctsäure und Cyclodextrin oder Cyclodextrinderivaten.

2. Einschlußverbindung aus (S)-Thioctsäure und Cyclodextrin oder Cyclodextrinderivaten.

3. Einschlußverbindung aus (R)-Dihydroliponsäure und Cyclodextrin oder Cyclodextrinderivaten.

4. Einschlußverbindung aus (S)-Dihydroliponsäure und Cyclodextrin oder Cyclodextrinderivaten.

5. Verwendung der Einschlußverbindungen gemäß einem der Ansprüche 1 - 4 zur Herstellung von Arzneimitteln.

6. Einschlußverbindungen gemäß einer der Ansprüche 1 - 4, dadurch gekennzeichnet,
daß als Cyclodextrin β-Cyclodextrin verwendet wird.

7. Verwendung der Einschlußverbindungen gemäß Anspruch 6 zur Herstellung von Arzneimitteln.

8. Einschlußverbindung aus Thioctsäure oder Dihydroliponsäure und mindestens einem Cyclodextrin aus der Gruppe der Methyl-, Ethyl- Carboxymethyl-, Hydroxyalkyl-, Dialkylaminoethyl-, Sulfoalkyl-, partiell methylierten Carboxyalkyl-Cyclodextrine, dimerisierten oder verzweigtkettigen Cyclodextrine vorliegt oder mit einem Überzug dieser Substanzen versehen ist.

9. Verwendung der Einschlußverbindung gemäß Anspruch 8 zur Herstellung von Arzneimitteln.

10. Verfahren zur Herstellung eines Arzneimittels, dadurch gekennzeichnet,
daß man Thioctsäure oder Dihydroliponsäure in Wasser suspendiert, Cyclodextrine hinzugibt und anschließend abkühlt und die Einschlußverbindung isoliert.

11. Verwendung des Granulats zur Herstellung einer Kautablette enthaltend als Wirkstoff Thioctsäure oder physiologisch unbedenkliche Salze von Thioctsäure, gegebenenfalls übliche Geschmacks-, Süß-, Aromastoffe sowie jeweils bezogen auf einen Gewichtsteil
Thioctsäure
0.001 - 1 Gewichtsteile eines Bindemittels
0.001 - 0.1 Gewichtsteile eines Fließhilfsmittels.

12. Verwendung des Granulats zur Herstellung einer Brausetablette enthaltend als Wirkstoff Thioctsäure oder physiologisch unbedenkliche Salze von Thioctsäure, gegebenenfalls übliche Geschmacks-, Süß-, Aromastoffe sowie jeweils bezogen auf einen Gewichtsteil
Thioctsäure
0.001 - 1 Gewichtsteile eines Bindemittels
0.001 - 0.1 Gewichtsteile eines Fließhilfsmittels
0.05 - 30 Gewichtsteile einer üblichen physiologisch unbedenklichen Brausemischung.

13. Granulat, Kau- oder Brausetablette nach Anspruch 11 und 12,
dadurch gekennzeichnet,
daß Thioctsäure in Form einer Einschlußverbindung mit Cyclodextrinen oder Cyclodextrinderivaten vorliegt.
